# EUROPEAN PATENT APPLICATION

(11) **EP 1 767 154 A1**
(43) Date of publication of application: **28.03.2007**
(21) Application number: 05758267.8
(22) Date of filing: 11.07.2005
(51) Int. Cl.: A61B 10/00

(54) **METHOD FOR ACQUIRING BIOINFORMATION USING MILLIMETER-WAVE BAND ELECTROMAGNETIC WAVE, DEVICE FOR ACQUIRING AND DISPLAYING BIOINFORMATION**

(30) Priority: 09.07.2004 JP 2004203526; 09.07.2004 JP 2004203527
(71) Applicant: Intellectual Property Bank Corp., 1-21-19, Toranomon, Minato-ku Tokyo 1050001 (JP)
(72) Inventor: IWAMATSU, Seiichi, Minato-ku, Tokyo 1050001 (JP); MARUI, Tomohiro, Minato-ku, Tokyo 1050001 (JP); KAWAGUCHI, Nobuaki, Minato-ku, Tokyo 1050001 (JP); SHINOZAKI, Makoto, Minato-ku, Tokyo 1050001 (JP)
(74) Representative: Grünecker, Kinkeldey, Stockmair & Schwanhäusser Anwaltssozietät
(86) International application number: PCT/JP2005/012757
(87) International publication number: WO 2006/006559

(57) **Abstract**

A method and device for extracting medically effective information by applying a millimeter-wave band electromagnetic wave to an organism and analyzing transmission, reflection and spontaneous radiation signals. The method and display comprise an acquiring step or means in which a database is so prepared in advance that from the transmission, reflection and spontaneous electromagnetic wave data measured in a state that an organism is irradiated with an electromagnetic wave having wavelength components the wavelengths of which are 6 to 14 mm and a state that the organism is not irradiated, electromagnetic characteristics at the organism surface and in the organism is categorized and organized by actual condition of the organism constituent element, and the actual condition information on the organism constituent element of the subject is acquired according to the database information from the transmission, reflection, and spontaneous electromagnetic wave data measured in a state that the organism, the subject, is irradiated with a similar electromagnetic wave and a state that the organism is not irradiated; a step of associating the position information on the measurement portion of the subject with three-dimensional meridians and meridian points; and an image displaying step.

## Description

### TECHNICAL FIELD

The present invention is an electromagnetic wave image analysis method and apparatus for living tissue. The electromagnetic wave image analysis method and apparatus are provided with two or more of at least one of the following analysis means: analysis means having means for applying an electromagnetic wave of millimeter wave range and means for receiving the transmission thereof, the reflection thereof, and spontaneous radiation; and analysis means not having means for applying an electromagnetic wave and composed only of reception means for receiving a spontaneous electromagnetic wave from a living tissue serving as a specimen. In the electromagnetic wave image analysis method and apparatus, signals from the abovementioned reception means are synthesized and image-processed by means of a computer having an appropriate interface to thereby be displayed, and thus abnormalities, a three-dimensional structure, a cross-sectional structure, a surface structure, or the like of the living tissue is displayed as an image. Hence, the electromagnetic wave image analysis method and apparatus are applied to observation of tissue of a human, an animal, or the like and are applied to early diagnosis, preventive diagnosis, treatment, or the like of various kinds of diseases.

### BACKGROUND ART

Conventionally, medical image diagnostic methods and apparatus have become widely available, such as an ultrasonic echo diagnostic method and apparatus, an X-ray tomography and an apparatus therefor (X-ray CT), a magnetic resonance method and apparatus (MRI), and a positron emission tomography and an apparatus therefor (PET).

Meanwhile, electromagnetic waves include not only so-called radio waves employed in radar and communications but also light and X-rays (Roentgen rays) each having a much higher frequency than the radio waves. Medical measurement utilizing X-rays is well known, and also a medical measurement technique utilizing the region of light and terahertz (THz) region is known (see Patent Documents 1, 2, and 3).

Of course, medical measurement utilizing the region of so-called radio waves is also known (see Patent Documents 4, 5, and 6). In particular, medical measurement utilizing a UWB (Ultra Wide Band) electromagnetic wave as the electromagnetic wave is also known (see Patent Documents 7, 8, and 9). Here, the ultra wide band electromagnetic wave is an electromagnetic wave having a transmission bandwidth extending 25% or more or 1.5 GHz or more from the center frequency of a signal. Patent Document 9 describes that the reflection wave of oscillated electromagnetic pulses is received and a medically useful image is obtained from the received signal to utilize the image for diagnosis. Furthermore, Patent Document 12 discloses a method and apparatus for evaluating the presence and absence of abnormalities in a female breast, a genital organ, and pathological abnormalities such as cancer for various living tissues in a human, an animal, and the like. Specifically, an electromagnetic wave having a specific frequency (430 to 480 MHz and multiples thereof) is applied, and absorption lines and frequency shifts generated by the application are observed by an electromagnetic spectrum analyzer to obtain information about anisotropy in a living tissue.

In general, only a small amount of data for organisms has been accumulated in the electromagnetic wave range of 10 GHz or more. On the other hand, since electrical characteristic data for cancerous cells and normal cells has been accumulated in a MHz band to about 10 GHz (order of "m" to "cm" in wavelength), medical diagnosis as in Patent Documents 5 and 6 is available. However, at frequencies over 10 GHz, order of "mm" in wavelength, data for organisms has not yet been fully provided. Hence, these conventional medical image diagnostic methods and apparatus have problems such as low resolution, giving radiation damage to living tissue, long diagnostic time, and high diagnostic cost due to high apparatus cost. In particular, as an example, in the exemplary case of cancer diagnosis, cancer detected by the above currently available medical diagnostic method and apparatus is often in intermediate or late stage. In addition to this, although the key of treatment is to detect initial variations early, it is very difficult to realize the early detection. The resolution of the above medical diagnostic methods and apparatus is several mm even in the smallest case. Thus, it has been very difficult to detect early initial variations of about several µm to about 1 mm.

Meanwhile, a technology is known in which a millimeter wave radar apparatus is employed to improve detection accuracy for an observation object. For example, Patent Document 11 is intended for observing a vehicle driving on a road, for detecting a mine in the ground, or for prospecting resources. However, the utilization of a millimeter electromagnetic wave for medical image imaging has not been disclosed since data for organisms has not yet fully provided as described above.

The above methods for acquiring bioinformation are a method in which the bioinformation is "actively" measured from transmission electromagnetic wave data and reflection electromagnetic wave data which are measured with an electromagnetic wave applied to an organism. On the other hand, a method is also known in which bioinformation is acquired from spontaneously radiated electromagnetic wave data which is "passively" measured with no electromagnetic wave applied to an organism. The spontaneously radiated electromagnetic wave is electromagnetic wave radiation in the infrared region, so-called a hot wave, and thus information depending on the temperature of an organism is obtained. Known surface temperature distribution imagers such as a thermograph and a thermoviewer utilize the spontaneously radiated electromagnetic wave. These imagers are regarded as an information imaging apparatus for the surface temperature of an organism.

Patent Document 10 relates to an imaging apparatus for a spontaneously radiated electromagnetic wave and discloses a method for knowing metabolic activity in a body and the presence of a tumor by obtaining temperature information inside an organism from radiation intensity data of infrared rays serving as a spontaneously radiated electromagnetic wave. In the above, a temperature transmission phenomenon in an organism is approximated by replacing it with an electrical circuit. Furthermore, by means of the electrical circuit model, information about the position of a heat source in an organism is obtained from the spontaneously radiated electromagnetic wave data of the organism in an inverse manner to thereby output a heat source distribution image. However, in the above invention, only thermal variations on the surface of a body are known, and a three-dimensional structure cannot be displayed as an image.

Patent Document 13 (Japanese Patent Laid-Open Publication No. Hei 10-192282) relates to an apparatus for knowing the condition of health of an organism by taking an image of a radiated reflection or transmission electromagnetic wave caused by applying an electromagnetic wave to a tooth or a bone or by taking an image of a radiated electromagnetic wave without a trading company. The exemplified electromagnetic wave employed therein is infrared rays. However, ultraviolet rays, visible rays, radio waves, X-rays, γ-rays, or the like may by employed, and these rays may be coherent or incoherent. No limitation is imposed on the electromagnetic wave to be employed. Furthermore, there is not any mention of how the effectiveness of the electromagnetic wave varies depending on the type thereof.

In the invention of Patent Document 14 (Japanese Patent Laid-Open Publication No. 2002-248088), an ultrahigh frequency (UHF) electromagnetic wave is radiated toward a first meridian point, and the electromagnetic wave radiated from a second meridian point is detected, whereby the conditions of an organ are diagnosed by means of the conductivity of signals or the permittivity between the two meridian points. In the above invention, the measurement is performed between the two points, and thus the measurement is not performed on the entire human body two-dimensionally or three-dimensionally.

The invention of Patent Document 15 (Japanese Patent Laid-Open Publication No. 2003-294535) is an apparatus and method for measuring the temperature of a measurement site by receiving an electromagnetic wave radiated from a human body, determining the conductivity or permittivity of the measurement site, and converting the conductivity or the permittivity to temperature. However, the measurement is performed only on a specific site of a human body, and thus the measurement is not performed on the entire human body two-dimensionally or three-dimensionally.

Furthermore, in Patent Document 16 (Japanese Patent Laid-Open Publication No. 2001-14446), Patent Document 17 (Japanese Patent Laid-Open Publication No. 2004-180932), Patent Document 18 (Japanese Patent Laid-Open Publication No. 2003-190101), and the like, a method has been disclosed in which images obtained through a plurality of medical imaging apparatus are combined for the same site.

Patent Document 1: Japanese patent No. 1911906, "Optical tomographic image visualizing apparatus", Japan Science and Technology Corporation, et al.

Patent Document 2: Japanese patent No. 3365397, "Multichannel optical measurement apparatus", Shimadzu Corporation.

Patent Document 3: International patent application No. WO00/50859, "Method and apparatus for TeraHertz Imaging", Toshiba Research Europe Ltd.

Patent Document 4: United States Patent No. 6448788, "Fixed array microwave imaging apparatus and method", Microwave Imaging System Technologies, Inc.

Patent Document 5: United States Patent No. 6421550, "Microwave discrimination between malignant and benign breast tumors", INTERSTITIAL INC.

Patent Document 6: United States Patent No. 6061589, "Microwave antenna for cancer detection system", INTERSTITIAL INC.

Patent Document 7: United States Patent No. 5361070, "Ultra-wideband radar motion sensor", Regents of the University of California.

Patent Document 8: United States Patent Application Laid-Open No. 2003/0090407, "Ultra-wideband imaging system", Santhoff, John H.

Patent Document 9: International patent application No. WO01/18533, "Radar Apparatus for Imaging and/or Spectrometric Analysis and Methods of Performing Imaging and/or Spectrometric Analysis of a Substance for Dimensional Measurement, Identification and Precision Radar Mapping" Stove George Collin.

Patent Document 10: United States Patent No. 6023637, "Method and apparatus for thermal radiation imaging", Liu, et al.

Patent Document 11: Japanese Patent Laid-Open Publication No. 2003-299066, "Image processing apparatus and method therefor", Matsushita Electric Industrial Co., Ltd.

Patent Document 12: Published Japanese translation of PCT international application No. 2003-530902, "Electromagnetic analyzer of anisotropy in chemically organized systems", VEDRUCCIO, Clarbruno.

Patent Document 13: Japanese Patent Laid-Open Publication No. Hei 10-192282, "Diagnostic apparatus for organism", Egawa Co., Ltd.

Patent Document 14: Japanese Patent Laid-Open Publication No. 2002-248088, "Apparatus and method for acquiring data for diagnosing organism utilizing ultrahigh frequency signal", Samsung Electronics Co., Ltd.

Patent Document 15: Japanese Patent Laid-Open Publication No. 2003-294535, "Noninvasive organism temperature measurement apparatus and method therefor", Samsung Electronics Co., Ltd.

Patent Document 16: Japanese Patent Laid-Open Publication No. 2001-14446, "Medical image processing apparatus", TOSHIBA CORPORATION and another company.

Patent Document 17: Japanese Patent Laid-Open Publication No. 2004-180932, "Computer aided diagnostic apparatus",

### TOSHIBA CORPORATION.

Patent Document 18: Japanese Patent Laid-Open Publication No. 2003-190101, "Diagnostic apparatus for organism", Konica corporation.

### DISCLOSURE OF THE INVENTION

### PROBLEMS TO BE SOLVED BY THE INVENTION

As in Patent Documents 1 to 12, there have been known the following methods and apparatus: the methods and apparatus for extracting medically useful information by analyzing transmission, reflection, and spontaneous radiation signals obtained by applying to an organism various electromagnetic waves including light; and the methods and apparatus for extracting medically useful information by analyzing a spontaneous electromagnetic wave of an organism. However, in order to obtain more useful information, it is effective to employ a millimeter-wave band electromagnetic wave. The present invention provides a method and apparatus for extracting medically useful information by applying an electromagnetic wave of millimeter wave region to an organism and analyzing the transmission, reflection, and spontaneous radiation signals thereof.

### MEANS FOR SOLVING THE PROBLEMS

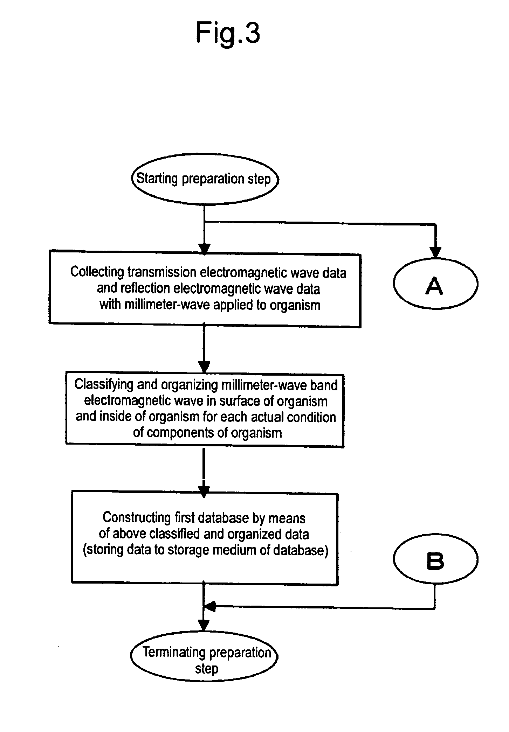

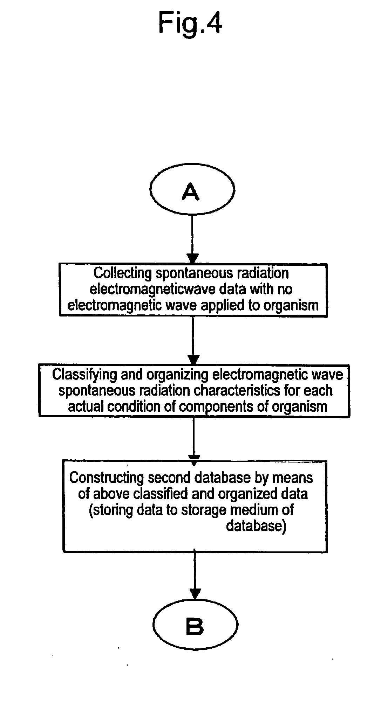

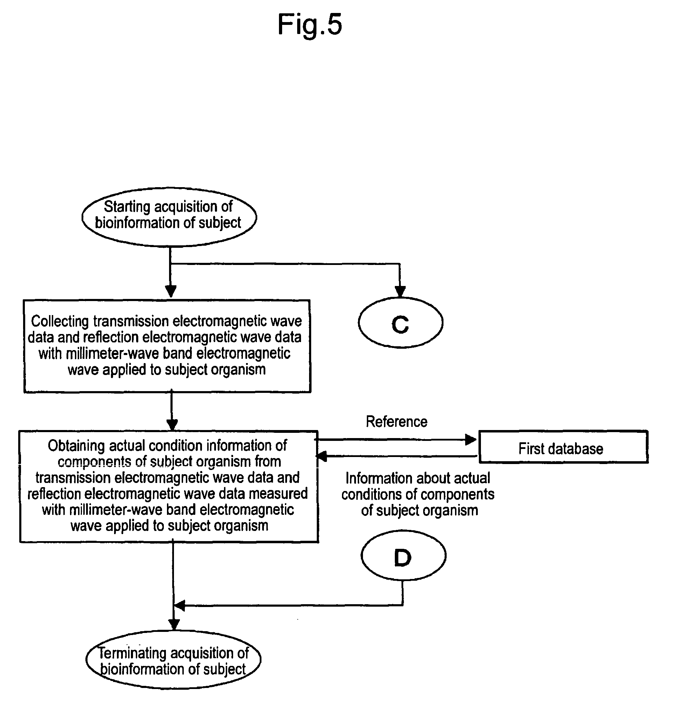

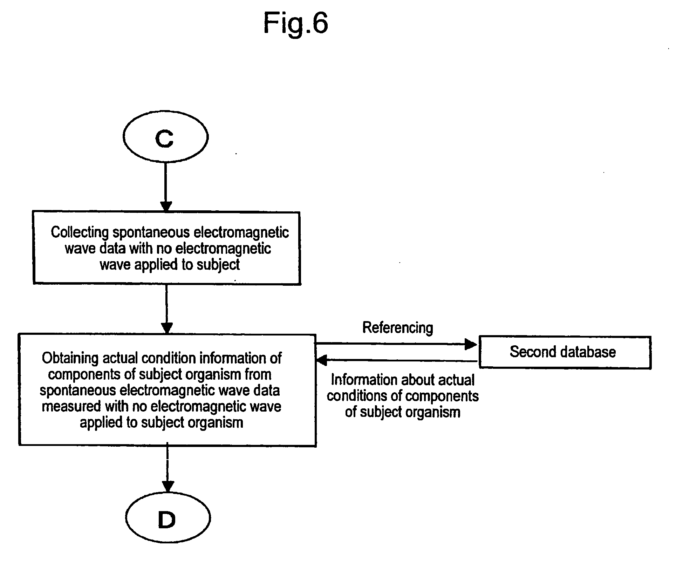

Accordingly, the present invention is a method for acquiring bioinformation. In this method, a first database is prepared in advance by classifying and organizing, for each of actual conditions of components of an organism, millimeter-wave band electromagnetic wave characteristics of a surface of the organism and an inside of the organism which characteristics contained in transmission electromagnetic wave data and reflection electromagnetic wave data measured with a millimeter-wave band electromagnetic wave applied to the organism (see Fig. 3). In addition, a second database is prepared in advance by classifying and organizing, for each of actual conditions of the components of the organism, electromagnetic wave spontaneous radiation characteristics of the organism which characteristics are contained in spontaneous radiation electromagnetic wave data measured with no electromagnetic wave applied to the organism (see Fig. 4). Furthermore, this method has: a step of acquiring, based on the information in the first database, actual condition information of components of a subject organism from transmission electromagnetic wave data and reflection electromagnetic wave data measured with a millimeter-wave band electromagnetic wave applied to the subject organism (see Fig. 5); and a step of acquiring, based on the information in the second database, actual condition information of the components of the subject organism from spontaneous radiation electromagnetic wave data measured with no electromagnetic wave applied to the subject organism (see Fig. 6). In the present description, the "actual conditions" mean a measured manner related to the physical position, hardness, dimensions, and the like of an organism.

Fig. 3 is an explanatory diagram of a flow for preparing the first database in which the millimeter-wave band electromagnetic wave characteristics of the inside of an organism are classified and organized for each of the actual conditions of the components of the organism. Fig. 4 is an explanatory diagram of a flow for preparing the second database in which the electromagnetic wave spontaneous radiation characteristics of the organism are classified and organized for each of the actual conditions of the components of the organism. Fig. 5 is an explanatory diagram of a flow of the step of acquiring, based on the information in the first database, the actual condition information of components of a subject organism from transmission electromagnetic wave data and reflection electromagnetic wave data measured with a millimeter-wave band electromagnetic wave applied to the subject organism. Fig. 6 is an explanatory diagram of a flow of the step of acquiring, based on the information in the second database, the actual condition information of components of a subject organism from spontaneous radiation electromagnetic wave data measured with no electromagnetic wave applied to the subject organism.

The preferred millimeter-wave band electromagnetic wave is an electromagnetic wave having an electromagnetic wave component of 6 mm to 14 mm, and the most preferable wavelength is 8 mm (37.5 GHz). Preferably, this electromagnetic wave is applied to an organism as a UWB (Ultra Wide Band) electromagnetic wave. This is because, since the energy at individual frequencies can be reduced by employing an ultra wide band electromagnetic wave, deleterious effects of an electromagnetic wave having a specific frequency on an organism can be avoided. Preferably, the electromagnetic wave energy density given to the surface of an organism is 40 mW/cm² or less.

An ultra wide band electromagnetic wave is generated through a plurality of repeated electromagnetic pulses which are continuous in time. Specifically, it is preferable that the millimeter-wave band electromagnetic wave of a present aspect have a bandwidth of 1.5 GHz or more and have at least an electromagnetic wave component having a wavelength of 6 mm to 14 mm, and that the millimeter-wave band electromagnetic wave characteristics of the surface of an organism and the inside of the organism contain at least the characteristics for an electromagnetic wave having a wavelength of 6 mm to 14 mm. Furthermore, it is preferable that the millimeter-wave band electromagnetic wave of the present aspect be generated through a plurality of repeated electromagnetic pulses which are continuous in time, that the pulse time width of the electromagnetic pulses be 0.1 to 10 nanoseconds, and that the repetition time interval of the plurality of electromagnetic waves be 0.1 to 10 microseconds.

Here, the electromagnetic wave having an electromagnetic wave component of 6 mm to 14 mm has the following three types of effectiveness: (1) As bioinformation, transmission electromagnetic wave data and reflection electromagnetic wave data in any wavelength region are effective which are obtained by applying an electromagnetic wave having an electromagnetic wave component of 6 mm to 14 mm for preparing the first database. (2) As bioinformation, spontaneous radiation electromagnetic wave data in the wavelength range of 6 mm to 14 mm is effective which is measured with no electromagnetic wave applied to an organism for preparing the second database. (3) Spontaneous radiation emerges later in time after the electromagnetic wave having an electromagnetic wave component of 6 mm to 14 mm is applied, and electromagnetic wave data contained in the spontaneous radiation and having the wavelength range of 6 mm to 14 mm is effective as bioinformation.

In (3), an electromagnetic reaction in an organism is induced by the application of the electromagnetic wave having an electromagnetic wave component of 6 mm to 14 mm and serving as a cue (trigger), whereby the spontaneous radiation in the wavelength range of 6 mm to 14 mm is induced. Therefore, (3) is included in (1) in a broad sense. As a mater of course, in order to utilize the effectiveness of the above (1), means for receiving an electromagnetic wave in an arbitrary (or a specific) wavelength range is required. Also, as a matter of course, in order to utilize the effectiveness of the above (2) and (3), means for receiving an electromagnetic wave of 6 mm to 14 mm is required.

In order to acquire a lot of bioinformation, the following configurations are conceivable: a configuration having a plurality of fixed radiation sources which radiate a millimeter-wave band electromagnetic wave toward an organism from respective different fixed positions; and a configuration in which a millimeter-wave band electromagnetic wave radiation source is a moving radiation source which is mounted on moving means and radiates an electromagnetic wave toward an organism from different positions while moving. In any of the above configurations, it is desirable that information indicating the positional relation (a direction and a distance) at the application of the electromagnetic wave toward an organism can be discriminated after reception. Therefore, it is desirable that the application be performed with the positional information of the millimeter-wave band electromagnetic wave generation source embedded in (superposed on) the radiation electromagnetic wave itself. This can be realized through an encryption technology of an electromagnetic wave. The cipher is decrypted after reception, and thus the positional information of the applied electromagnetic wave having generated the received electromagnetic wave is obtained. It is desirable that the superposed cipher be a digital cipher which does not affect the acquisition of bioinformation. In radar detection technology, a technique is known in which a digital cipher is superposed on a radiation radio wave and is decrypted in a reception side for use.

Specifically, it is preferable that the millimeter-wave band electromagnetic wave be generated at different fixed positions or at a position varying with time, and that information about the generation position be encrypted and superposed on the electromagnetic waves. Furthermore, it is preferable that millimeter-wave band electromagnetic wave generation information be added to the millimeter-wave band electromagnetic wave characteristics of the surface of an organism and the inside of the organism, the millimeter-wave band electromagnetic wave generation information being obtained by decrypting, based on an encryption method employed for encrypting and superposing the abovementioned information about the generation positions, transmission electromagnetic wave data and reflection electromagnetic wave data measured with a millimeter-wave band electromagnetic wave applied.

The transmission electromagnetic wave data and reflection electromagnetic wave data measured by applying to an organism the millimeter-wave band electromagnetic wave generated simultaneously at different fixed positions or generated at a plurality of timings at a position varying with time are converted to two-dimensional or three-dimensional organism image information by means of a known synthetic aperture radar (SAR) technique. In this image information generation, the abovementioned millimeter-wave band electromagnetic wave generation position information obtained by the aforementioned cipher decryption is utilized.

If preset data for positions of tissues and organs in an organism is provided when image information of an organism is obtained, processing efficiency of image conversion is improved. Preferred preset positions are positions corresponding to "meridian points" and "meridians" (positions corresponding to acupoints in acupuncture) used in oriental medicine. Preferably, these positions are employed as preset data and serve as temporary positions indicating positional relation when the positions of tissues and organs in an organism are specified, or these positions are utilized as intersections of a mesh for image analysis. The meridian points and the meridians serve as three-dimensional relative position coordinates of a body and at the same time serve as measurement positions for measuring bioinformation in order to know health conditions of a subject for the measurement. A key to know whether or not the conditions of a subject for measurement are normal can be obtained by accumulating, in a database in advance, bioinformation values at meridian points and meridians for a normal case, measuring bioinformation values at the corresponding meridian points and meridians of the subject for measurement, and comparing the measured values with the normal values. For example, in the case where the balance of a meridian function of a small intestine is lost, a difference occurs between a normal small intestine meridian line and a small intestine meridian line drawn by means of the medical diagnostic apparatus according to the present invention. The larger the difference is, the worse the malfunction condition is. Thus, desirably, an intensive examination is carried out for internal organs and tissue organs associated with the small intestine meridian. However, when the function is normal, the difference between the measured meridian line and the normal meridian line is small, or the measured meridian line coincides with the normal meridian line. This means that medical conditions are normal. In order to solve the abovementioned problems, the electromagnetic wave medical diagnostic method and apparatus according to the present invention is characterized by having two or more of at least one of the following analysis method: an analysis method having electromagnetic wave radiation means and electromagnetic wave reception means; and an analysis method not having electromagnetic wave radiation means and composed only of means for receiving a spontaneous electromagnetic wave from a living tissue serving as a specimen. In addition, the method and apparatus is characterized by employing means for synthesizing and image-processing signals from the abovementioned analysis methods by means of a computer having an appropriate interface to thereby display an image of a three-dimensional structure, cross-sectional structure, surface structure, or the like of an affected part. Furthermore, in order to solve the abovementioned problems, the electromagnetic wave medical diagnostic method and apparatus according to the present invention is characterized by employing means for processing signals or the like from the abovementioned two or more analysis methods by means of a digital signal processor or the like through respective appropriate interfaces or the like, for introducing the signals or the like to a digital signal processor or the like having a storage unit and an analysis unit and equipped with software for diagnosis, for synthesizing both the signals and performing analytical diagnostic computational processing, for inputting signals outputting the results of the computational processing to a digital signal processor or the like to perform processing such as image processing, and for outputting to an image display apparatus or the like for displaying.

### EFFECTS OF THE INVENTION

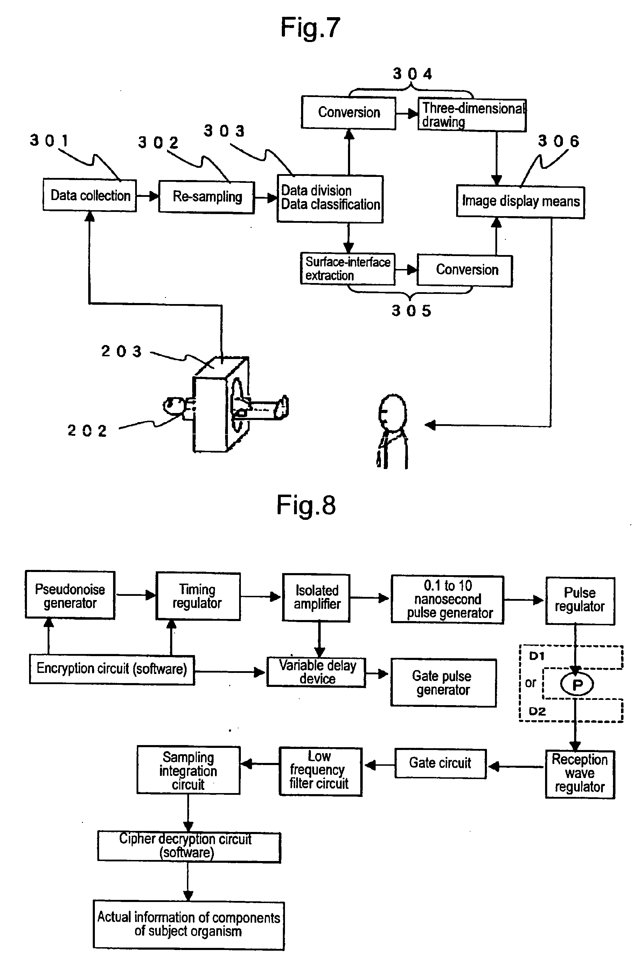

In contrast to bioinformation obtained by a bioinformation acquisition method using conventional electromagnetic wave region, qualitatively and quantitatively better useful bioinformation can be efficiently obtained by employing a millimeter-wave band electromagnetic wave, and thus more appropriate medical diagnosis is available. Furthermore, an image diagnostic method and apparatus are provided which enable high speed diagnosis at high resolution without causing radiation damage, thereby providing an effect of achieving low diagnosis cost. Furthermore, in an example of applying the present invention to cancer diagnosis, the diagnosis can be performed more reliably by means of diagnoses such as a high resolution three-dimensional image diagnosis, a high resolution cross-sectional image diagnosis, and a diagnosis by displaying an affected part with a color or the like applied thereto. Thus, an affected part in initial stage can be detected early, thereby providing an effect of facilitating treatment. Fig. 7 is an explanatory diagram for providing bioinformation for diagnosis by a doctor by means of an apparatus of an aspect of the present invention.

### BRIEF DESCRIPTION OF THE DRAWINGS

Fig. 1 is an explanatory diagram of a bioinformation acquisition apparatus (similar to an ultrasonic CT tomographic image display apparatus) of an aspect of the present invention, the apparatus employing electromagnetic wave radiation means and electromagnetic wave reception means contacting the surface of an organism.
Fig. 2 is an explanatory diagram of a bioinformation acquisition apparatus (similar to an X-ray CT tomographic image display apparatus) of an aspect of the present invention, the apparatus employing electromagnetic wave radiation means and electromagnetic wave reception means not contacting the surface of an organism.
Fig. 3 is an explanatory diagram of a flow for preparing a first database in which millimeter-wave band electromagnetic wave characteristics of the inside of an organism are classified and organized for each of the actual conditions of components of the organism.
Fig. 4 is an explanatory diagram of a flow for preparing a second database in which electromagnetic wave spontaneous radiation characteristics of an organism are classified and organized for each of the actual conditions of components of the organism.
Fig. 5 is an explanatory diagram of a flow of a step of acquiring, based on the information in the first database, the actual condition information of components of a subject organism from the transmission electromagnetic wave data and reflection electromagnetic wave data measured with a millimeter-wave band electromagnetic wave applied to the subject organism.
Fig. 6 is an explanatory diagram of a flow of a step of acquiring, based on the information in the second database, the actual condition information of the components of a subject organism from spontaneous radiation electromagnetic wave data measured with no electromagnetic wave applied to the subject organism.
Fig. 7 is an explanatory diagram of providing bioinformation for diagnosis by a doctor by means of an apparatus of an aspect of the present invention.
Fig. 8 is an explanatory diagram of encryption means of a millimeter-wave band electromagnetic wave and decryption means thereof.
Fig. 9 is a diagram of a configuration example of millimeter-wave band electromagnetic wave radiation means.
Fig. 10 is a diagram of a configuration example of electromagnetic wave reception means.
Fig. 11 is a block diagram of main portions, illustrating an example of the radar medical diagnostic method and apparatus according to the present invention.
Fig. 12 is a schematic diagram of main portions, illustrating an application example of the radar medical diagnostic method and apparatus according to the present invention.
Fig. 13 is a flowchart for producing a three-dimensional image.
Fig. 14 is a flowchart for producing two types of three-dimensional images.
Fig. 15 is a comparison of required computational time between an MB method and an MC method.
Fig. 16 is a block diagram of a DMC method.
Fig. 17 is a block diagram of a VOMI system.
Fig. 18 is a schematic diagram of main portions of a human body, illustrating an example of gray scale tree-dimensional image display by means of the radar medical diagnostic method and apparatus according to the present invention.

### DESCRIPTION OF THE SYMBOLS

D1: Electromagnetic wave reception means of a type in which the means contacts the surface of an organism (similar to an ultrasonic CT tomographic image display apparatus).

D2: Electromagnetic wave reception means not contacting the surface of an organism (similar to an X-ray CT tomographic image display apparatus).

M: Means for linearly moving a subject organism placed thereon in an examination zone of D2.

P: Subject organism.

Rad: Means for radiating a millimeter-wave band electromagnetic wave (radiation antenna).

Rec: Means for receiving an electromagnetic wave caused by reflection/transmission of a millimeter-wave band electromagnetic wave from/through a subject organism or means for receiving an organism spontaneous radiation electromagnetic wave (reception antenna).

DSP: Digital signal processor.
101: Analysis means unit provided with electromagnetic wave radiation means and electromagnetic wave reception means.
102: Analysis means unit not having electromagnetic wave application means and composed only of means for receiving a spontaneous electromagnetic wave from living tissue serving as a specimen.
103: Image processing unit.
201: Diagnostic bed.
202: Human body.
203: Reception means.
204: Scan direction.
301: Data collection unit.
302: Re-sampling unit.
303: Separation-classification unit.
304: Polygon unit (conversion unit and three-dimensional drawing unit).
305: Polygon unit (surface extraction unit and conversion unit).
306: Display unit.
501: Master unit.
502: Slave unit.
601: Data collection unit.
602: Data processing unit.
603: Viewpoint conversion unit.
604: Beam tracing unit.
605: Body unit projection unit.
606: Viewpoint conversion unit.
607: Display unit.
701: Three-dimensional image data.
702: Intermediate surface display unit.
703: Digital three-dimensional image unit.
801: Data collection unit.
802: Data processing unit.
803: Data storage.
804: Preprocessing unit.
805: Viewpoint conversion unit.
806: Slice production unit.
807: Surface image production unit.
808: Three-dimensional image production unit.
809: Display unit.
810: Interactive unit.
811: Image conversion unit.

### BEST MODE FOR CARRYING OUT THE INVENTION

As the best mode for carrying out the present invention, the apparatus of the present invention will be described. The apparatus of the present invention is constituted by an electromagnetic pulse generator, a frequency regulator for an electromagnetic wave, an electromagnetic wave receiver, a signal processor, and the like. These can be implemented by use of a combination of known technologies. A sound wave receiver, an optical receiver, an electromagnetic wave receiver for infrared (temperature) region (a thermograph), or the like is employed as a receiver in accordance with need.

Furthermore, in the present aspect, an organism tomographic image similar to that of CT (computer tomography) or a three-dimensional solid image can be reconstructed and displayed by use of physical data values in the first and second databases as color and gray scale information, and this can easily implemented by means of known software and a known image display apparatus. Since complicated information about the inside of an organism is grasped, it is preferable that a tomographic image and a three-dimensional solid image of a subject organism be displayed in parallel. This can also be implemented by means of a known technology.

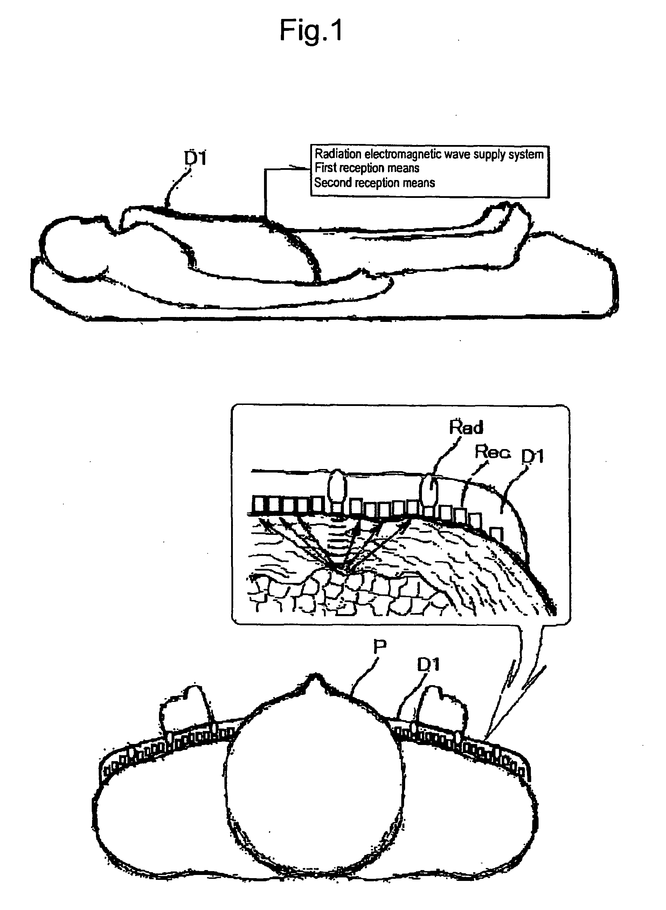

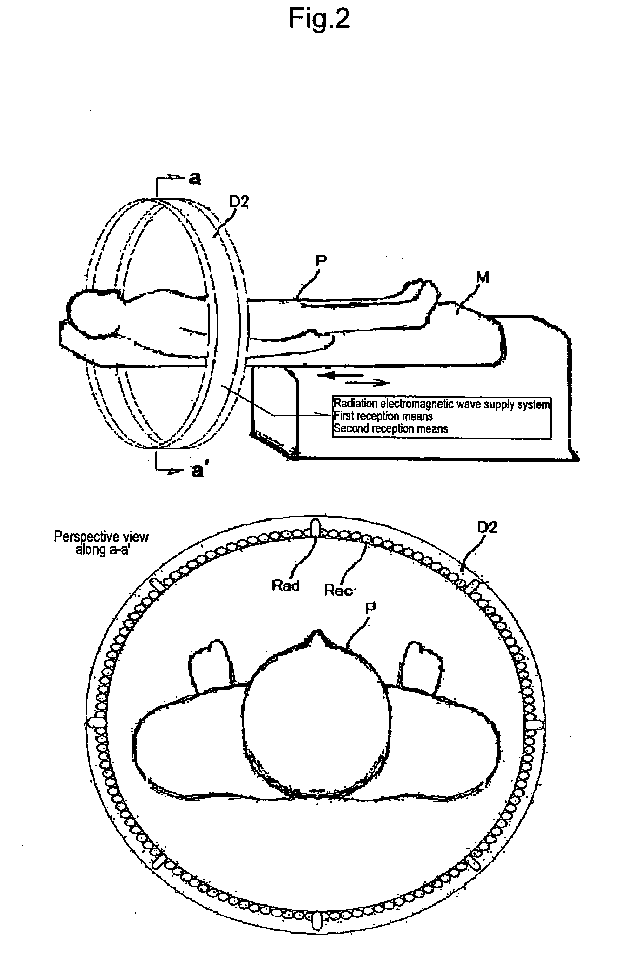

For the hardware devices (an electromagnetic wave generator and an electromagnetic wave receiver) constituting the present aspect, two implementations can be considered. One is a method in which an electromagnetic wave (sound wave) generator and an electromagnetic wave (sound wave) receiver are brought into direct contact with the surface of an organism as in an ultrasonic diagnostic apparatus. The other is a method in which an electromagnetic wave (X-ray) generator and an electromagnetic wave (X-ray) receiver are separated from an organism as in X-ray CT (Computer Tomography). For example, the generator and the receiver are arranged so as to surround the organism in a ring-like shape. The former is shown in Fig. 1, and the latter is shown in Fig. 2.

In Figs. 1 and 2, D1 is electromagnetic wave reception means of a type in which the means contacts the surface of an organism (similar to an ultrasonic CT tomographic image display apparatus), and D2 is electromagnetic wave reception means not contacting the surface of an organism (similar to an X-ray CT tomographic image display apparatus). Furthermore, M is means for linearly moving a subject organism placed thereon in an examination zone of the D2, and P is a subject organism. Rad is means for actively radiating an electromagnetic wave, and Rec is means for detecting a reflection/transmission electromagnetic wave of an electromagnetic wave radiated from/through the Red or means for detecting a spontaneous radiation electromagnetic wave measured with no millimeter-wave band electromagnetic wave applied to an organism. The Rec represents these two, or both the means for actively detecting the reflection/transmission electromagnetic wave and the means for passively detecting the spontaneous radiation electromagnetic wave. For simplicity, these two means are not separated in the figure.

The D2 in Fig. 2 is arranged in a ring-like shape over the entire 360 degrees. However, the D2 may be arranged partially, and measurement may be performed by rotating the arranged portion of the D2 around the center axis of the ring. Such a technique is known also in a CT (Computer Tomography) technology and can be easily implemented by following the CT technology.

The first and second databases of the present aspect are a general database constituted by a general storage apparatus for a computer and the like and are not a special database. When the databases are generated, it is preferable that the data be organized not only according to the intensity of transmission, reflection, spontaneous radiation signals of an electromagnetic wave in an organism but also by means of a time domain (a frequency domain) and a phase domain. This can be implemented by means of a known signal processing technique and a known database construction technique.

A summary of the apparatus configuration is described. That is, the bioinformation acquisition apparatus has: a first database in which millimeter-wave band electromagnetic wave characteristics of a surface of an organism and an inside of the organism are classified and organized for each of actual conditions of components of the organism, the millimeter-wave band electromagnetic wave characteristics being contained in transmission electromagnetic wave data and reflection electromagnetic wave data measured with a millimeter-wave band electromagnetic wave applied to the organism; a second database in which electromagnetic wave spontaneous radiation characteristics of the organism are classified and organized for each of actual conditions of the components of the organism, the electromagnetic wave spontaneous radiation characteristics being contained in spontaneous radiation electromagnetic wave data measured with no electromagnetic wave applied to the organism; millimeter-wave band electromagnetic wave radiation means for radiating a millimeter-wave band electromagnetic wave toward a subject organism; first reception means for receiving an electromagnetic wave from the subject organism irradiated with the millimeter-wave band electromagnetic wave; second reception means for receiving an electromagnetic wave spontaneously radiated from the subject organism; and means for acquiring actual condition information of the components of the subject organism based on the data obtained by the abovementioned first and second reception means and the information in the abovementioned first and second databases. (Here, the millimeter-wave band electromagnetic wave radiation means is the Rad in Figs. 1 and 2, and the first reception means and the second reception means are the Rec not separately illustrated in Figs. 1 and 2.)

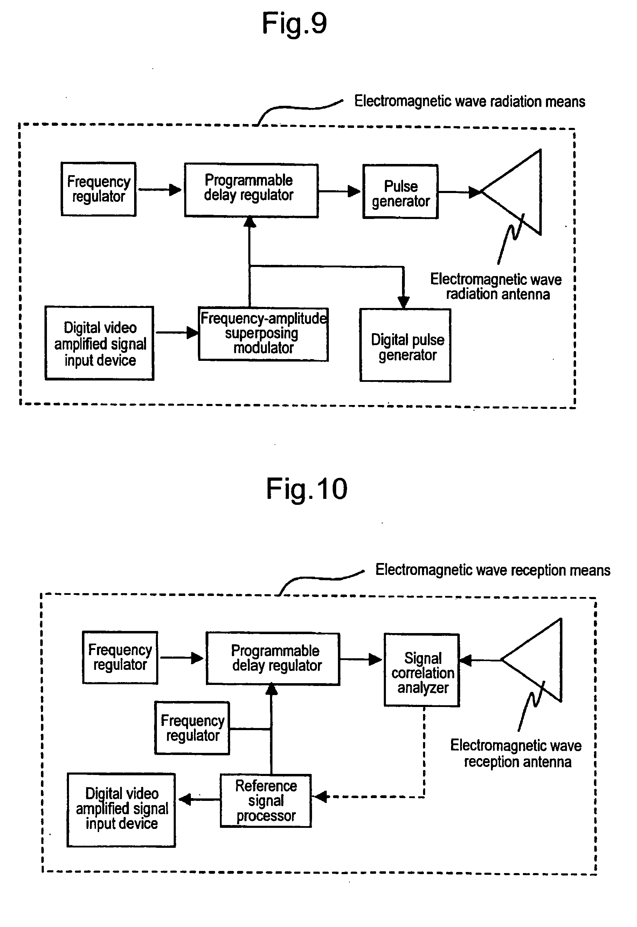

The millimeter-wave band electromagnetic wave radiation means has a radiation source which radiates an electromagnetic wave containing at least an electromagnetic wave component having a wavelength of 6 mm to 14 mm, and the first reception means has a receiver which receives an electromagnetic wave containing at least an electromagnetic wave component having a wavelength of 6 mm to 14 mm. Furthermore, the radiation source has an electromagnetic pulse generator which generates a plurality of repeated electromagnetic pulses which are continuous in time. The pulse time width of the electromagnetic pulses is 0.1 to 10 nanoseconds, and the repeated time interval of the plurality of electromagnetic waves is 0.1 to 10 microseconds. Configuration examples of these radiation means and reception means are shown in Figs. 9 and 10, respectively. A "programmable delay regulator" in Figs. 9 and 10 is related to encryption described next. The description of the other blocks in the figure is omitted.

When the millimeter-wave band electromagnetic wave radiation means is composed of a plurality of fixed radiation sources which radiate a millimeter-wave band electromagnetic wave from respective different fixed positions toward an organism as shown in Figs. 1 and 2, this radiation means has also electromagnetic wave encryption means for encrypting information of the fixed position of the radiation sources and superposing the encrypted information on the electromagnetic wave radiated from the abovementioned radiation sources. In addition, the first reception means has also cipher decryption means for decrypting received electromagnetic wave data based on the encryption method employed for encrypting and superposing the abovementioned information of the fixed position. Hence, the information indicating the positional relation (a direction and a distance) at the application of the millimeter-wave band electromagnetic wave to an organism is discriminated after reception and is utilized for grasping bioinformation. More specifically, for identifying the position of tissues and organs in an organism, and for determining the conditions of the tissues and organs in the organism, the direction and the distance of electromagnetic wave application serve as important conditions.

When the millimeter-wave band electromagnetic wave radiation means is a moving radiation source which is placed on moving means and radiates a millimeter-wave band electromagnetic wave toward an organism from different positions while moving (not shown in the figure, claim 9), this radiation means also has electromagnetic wave encryption means for encrypting positional information at the time of radiation and superposing the encrypted information on the electromagnetic wave radiated from the abovementioned moving radiation sources. In addition, the first reception means has also cipher decryption means for decrypting received electromagnetic wave data based on the encryption method employed for encrypting and superposing the abovementioned positional information at the time of electromagnetic wave radiation. Similar to the above, the information indicating the positional relation (a direction and a distance) at the application of the millimeter-wave band electromagnetic wave to an organism is discriminated after reception and is utilized for grasping bioinformation. Fig. 8 is an explanatory diagram of the encryption means and the decryption means of a millimeter-wave band electromagnetic wave. The description of the other blocks in Fig. 8 is omitted.

Hereinafter, the electromagnetic wave medical diagnostic method and apparatus according to an embodiment of the present invention are described in detail with reference to the drawings.

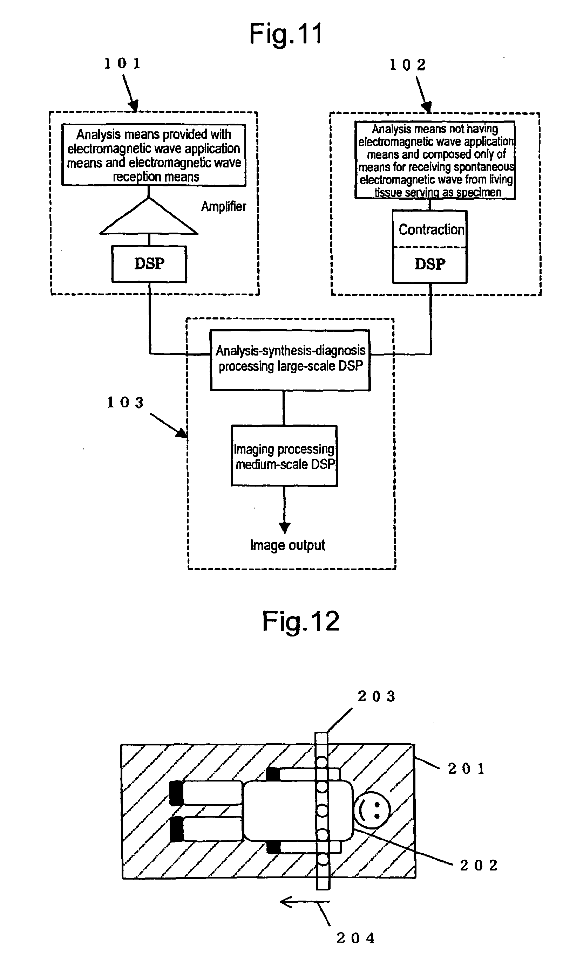

Fig. 11 is a block diagram of main portions, illustrating an example of the electromagnetic wave medical diagnostic method and apparatus according to the present invention.

The abovementioned medical diagnostic method and apparatus are composed of three blocks, i.e., an analysis means unit 101 provided with electromagnetic wave application means and electromagnetic wave reception means, an analysis means unit 102 not having electromagnetic wave application means and composed only of means for receiving a spontaneous electromagnetic wave from living tissue serving as a specimen, and an image processing unit 103 forming an image signal by synthesizing and analytically diagnosing signals from these two types of analysis means.

In the analysis means unit 101 provided with the electromagnetic wave application means and the electromagnetic wave reception means, signals from a synthetic aperture radar composed of a low power variable frequency oscillator unit of about 10 to 20 mW and an antenna unit are amplified by an amplifier, are processed by a small-scale digital signal processor (DSP) of about 32 bits which processor forms appropriate digital signals, and are sent to the image processing unit 103.

An example of a method and apparatus employed as the analysis means provided with the electromagnetic wave application means and the electromagnetic wave reception means is a synthetic aperture radar. In the synthetic aperture radar, a technology for a phased array antenna is applied.

In the phased array antenna, a plurality of microwave sensor antennas are employed as a group. A phase shifter is provided in each of the antennas, and the phases for the antennas are shifted stepwise to one another. In this manner, the phased array antenna serves as a group of antennas and is set so as to be focused on a point (a direction and a distance) in a narrowed beam. Since radio waves are mutually intensified at a point at which the phases coincide with each other, or at the focal point, measurement can be performed with high directivity and high resolution. The synthetic aperture radar is based on the phased array antenna. For example, for the case of a meteorological satellite, the synthetic aperture radar continuously collects signals in a line shape as the satellite moves, and converts the signals to measurements for a planar shape by means of data processing.

In the synthetic aperture radar, the resolution for a relatively large diameter radar can be obtained through a group of small sensors, and thus high resolution can be obtained. On the other hand, since the processing of the received signals requires a huge amount of computations, the synthetic aperture radar has been actively utilized in recent years where the performance of computers progresses, and has been mounted mainly on meteorological satellites and military aircrafts.

In the present invention, by utilizing the high resolution of the synthetic aperture radar, an initial variation (about several µm to about 1 mm) of a subject specimen can be detected early.

The analysis means unit 102 not having electromagnetic wave application means and composed only of the reception means for receiving a spontaneous electromagnetic wave from living tissue serving as a specimen serves as second reception means in the example. In this analysis means unit 102 in the example, signals from reception means composed of two or more infrared ray sensor group units containing a lens system for receiving infrared rays radiated from a subject specimen are processed by a small-scale digital signal processor (DSP) of about 32 bits to perform signal processing such as removal of various types of noise and smoothing the signals and to reduce the data size, whereby appropriate digital signals are formed and sent to the image processing unit 103.

In the image processing unit 103, by means of a large-scale digital signal processor (DSP) of about 256 bits which processor contains a storage unit and an analysis unit and has diagnostic software installed therein, the signals from the analysis means unit 101 provided with the electromagnetic wave application means and the electromagnetic wave reception means and the signals from the analysis means unit 102 not having electromagnetic wave application means and composed only of the means for receiving a spontaneous electromagnetic wave from living tissue serving as a specimen are synthesized and subjected to computational processing for analytical diagnosis at high speed. The signals subjected to the computational processing are subjected to image processing by means of a medium-scale digital signal processor (DSP) of about 64 bits, and the image processed signals are outputted to an image display unit.

The scale of the digital signal processors (DSP) is defined as large, medium, or small and is represented by 256, 64, or 32 bits. However, the scale is only exemplary and is not limited in the present invention.

In the present invention, the reception means provided with the electromagnetic wave application means is utilized for the purpose of detecting structural variations of tissue of a patient.

As an example of the analysis means not having electromagnetic wave application means and composed only of the means for receiving a spontaneous electromagnetic wave from living tissue serving as a specimen, an infrared ray sensor is applied thereto. The principle of the above reception means is derived from the viewpoint of oriental medicine, and thus the position, including the depth from body surface, of a focus is detected by measuring the temperature distribution of the body surface of a human.

In oriental medicine, for example, a determination is made that a tumor is likely to grow in circumstances in which the temperature of a lip rises. Furthermore, for example, when the temperature of a navel lowers, a determination is made that recovery after illness is not satisfactory. An infrared ray sensor or the like is utilized for measuring the temperature of body surface.

The method by means of an infrared ray sensor includes the following idea as a part thereof. Thermal radiation from the body surface of a human is measured by means of the infrared ray sensor to thereby obtain a temperature distribution diagram of the body surface. The estimation of the depth of a heat source or the position of an abnormal area of living tissue from the body surface is based on an assumption that a Gaussian distribution can be used for modeling the temperature distribution of the body surface. A half-value point is a point which bisects the area enclosed by a Gaussian distribution curve. When the half-value point can be found, the depth of the heat source can be found. The idea is based on the above assumption.

In the present invention, by employing one or two or more infrared ray sensors in the analysis means not having electromagnetic wave application means and composed only of the means for receiving a spontaneous electromagnetic wave from living tissue serving as a specimen, the position of a subject such as an affected part can be grasped more correctly.

The depth of an abnormal part of living tissue can be estimated by means of one infrared sensor. However, the position of a heat source can be identified more correctly by detecting the position of the heat source from different angles by means of a plurality of infrared ray sensors of the reception means.

In the method and apparatus according to the present invention, different variations in an affected area are detected by means of a plurality of types of analysis means employing different principles, i.e., the analysis means provided with the electromagnetic wave application means and the electromagnetic wave reception means and the analysis means not having electromagnetic wave application means and composed only of the means for receiving a spontaneous electromagnetic wave from living tissue serving as a specimen. Then, the detection results are subjected to computing and are multi-viewed, whereby diagnosis of the affected part can be performed more correctly.

Fig. 12 is a schematic diagram of main portions, illustrating an application example of the electromagnetic wave medical diagnostic method and apparatus according to the present invention.

A human body 202 serving as an example of a subject specimen is placed on a diagnostic bed 201, and reception means 203 such as a synthetic aperture radar scans the human body in a scan direction 104. Here, the reception means is employed as a generic term for the reception means provided with the electromagnetic wave application means and a method not having electromagnetic wave application means and composed only of the reception means for receiving a spontaneous electromagnetic wave from living tissue serving as a specimen.

The diagnostic bed 201 has dimensions of about 2 m in length and about 80 cm in width and is placed in a shield room which shields noises or the like such as electromagnetic wave noises and noises from outside environment or the like. A reflection plate reflecting an electromagnetic wave is placed in the diagnostic bed 201.

A living specimen, such as the human body 202, placed on the diagnostic bed 201 wears a hospital gown made of, for example, synthetic fiber, such as acrylic, polyester, or nylon, transmitting electromagnetic waves and infrared rays.

Two methods can be employed as the scanning method for the reception means 203. One is a method in which the human body 202 is stationary and the reception means moves. The other is a method in which the reception means 203 is stationary and the diagnostic bed 201 and the human body 202 move.

The collected three-dimensional image data is displayed as, for example, a transparent image. Examples of the method for displaying the transparent image include a maximum echo method, a minimum echo method, and an X-ray method. A three-dimensional structure, a cross-sectional structure, a surface structure, a coronary structure, or the like is drawn.

By means of the maximum echo method, a focus such as hepatic aneurysm can be detected by displaying a part producing a large echo. By means of the minimum echo method, a focus, such as sac tumor, located in a part producing a small echo can be detected. The X-ray method employs a display method providing a figure similar to that in an X-ray inspection, and the results are given as the gray scale average values of the results of the maximum echo method and the minimum echo method.

Fig. 7 is a flowchart of visualizing the signals collected by the reception means and is mainly describes the unit 103 of Fig. 11 in more detail. A portion corresponding to the diagnostic bed 201 shown in Fig. 12 is illustrated in a part of this figure.

This is a method for extracting and displaying an image of a subject affected part or a subject living organ from the inside of living tissue serving as a measurement subject such as a human body. A measurement subject such as an affected part or a living organ is determined by setting an appropriate threshold value.

Signals collected from the reception means 203 are received by a data collection unit 301. Then, a re-sampling unit 302 performs noise removal from the signals, unification of different formats for both the reception means into the same image file format, unification of the sizes of the voxels in both the reception means, unification of the space coordinates of the voxels, and the like. In a separation-classification unit 303, the signals are divided to different lines. The shapes of the measurement subject such as an affected part or a living organ are formed in a three-dimensionalizing processing unit 304 and in a polygonalizing processing unit 305. The three-dimensionalizing processing unit 304 employs a method for drawing the internal structure of a human body or the like serving as a measurement subject as if it were transparent. In the polygonalizing processing unit 305, the shape of an affected part, an organism, or the like is drawn by use of voxels having one certain threshold value. The images processed by the three-dimensionalizing processing unit 304 and the polygonalizing processing 305 are displayed in a display unit 306.

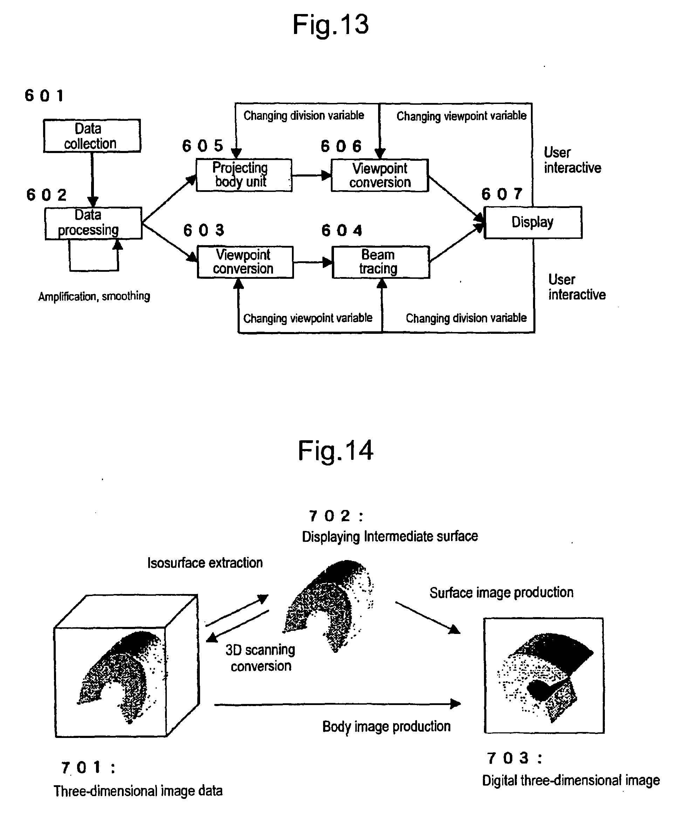

Fig. 13 is a flowchart for producing a three-dimensional image. The data from the reception means 203 is collected in a data collection unit 601. Subsequently, the data is sent to a data processing unit 602 which performs processing such as signal amplification and noise removal. Then, in a viewpoint conversion unit 603, the collected raw data is subjected to conversion to voxel values in a three-dimensional space, coordinate system conversion, conversion to a screen coordinate system, or the like.

In a beam tracing unit 604, processing according to a beam tracing method is performed. In the beam tracing method, it is assumed that a beam is emitted toward each point in a measurement subject from a viewpoint, and the color value and the opacity of a point on the beam are determined. The beam tracing method is based on a principle in which "all the results indicating what color of which point appears at the point are summed up."

A body unit projection method is a method for determining a shape of a solid by determining the color value and the opacity of each voxel. In a body unit projection unit 605, the transparency, the color values (RGB), the gradient of inclination, and the like are computed for each drawn point on an isosurface, and a gloss model indicating diffusion, reflection, transmission, absorption, scattering, and the like of light is computed according to a material to thereby produce a three-dimensional image composed of curved surfaces.

A viewpoint conversion unit 606 designates which affected part is paid attention according to the setting of the viewing angle, the expansion/retraction magnification, the threshold value for a measurement subject such as an affected part or a living organ. A drawn three-dimensional image is displayed on a display unit 607.

Fig. 14 describes two methods for producing a three-dimensional image. One method is referred to as surface image production and is the method described in Fig. 4. An isosurface is extracted from an original three-dimensional data 701 by use of a threshold value or an extreme. An image formed through the isosurface is referred to as an intermediate surface. In an intermediate surface display unit 702, the computations for light reflection, light transmission, and the like described in Fig. 4 are performed to form a curved image, and the image is displayed on a digital three-dimensional image unit 703.

The surface image production is suitable for measuring the volume, the area, the length, and the like of a subject. Since the computational load on hardware is low, the surface image production is currently mainstream.

In body image production, an image is directly drawn by use of the original three-dimensional data 701 without constructing an intermediate surface. The image is non-binary image having gradation. By drawing a translucent structure, the image can be represented more realistic and more intuitive. This is a method which has been received increasing attention in recent years.

### Example 1

Hereinafter, for the radar medical diagnostic method and apparatus of the present invention, a detailed description will be given of another Example according to Figs. 11 and 12.

An electromagnetic wave from an electromagnetic wave radiator of the analysis means provided with the electromagnetic wave application means and the electromagnetic wave reception means is a pulse electromagnetic wave having a duration of the applied electromagnetic wave of 0.1 nanoseconds to 10 nanoseconds and an interval between the pulses of 100 times to 10,000 times of the pulse duration and also is an electromagnetic wave having a single wavelength within the wavelength range of 6 mm to 12 mm. A reception sensor of the radar is a group of microwave sensor antennas. In order to reduce the load on a living specimen, the power of the radiator of the applied electromagnetic wave such as a variable wavelength microwave is lowered to the extent of not affecting the specimen and is about 10 kW or less. When the entire specimen is scanned, an electromagnetic wave of about 10 mW is applied. When only an affected part is scanned, an electromagnetic wave of about 20 mW is applied.

When a human body is employed as a specimen, a short scanning time of 10 to 30 seconds is employed. Particularly, when an affected part is scanned separately, a scanning time of about 10 seconds is enough. Since a detected wave at the reception means provided with the application means of the electromagnetic wave is a small signal, this signal is amplified by an amplifier and is then sent to a first stage digital signal processor (DSP).

The analysis means not having electromagnetic wave application means and composed only of the reception means for receiving a spontaneous electromagnetic wave from living tissue serving as a specimen can also receive a very low power spontaneous electromagnetic wave from an organism.

Analysis means not having infrared ray application means and composed only of means for receiving a spontaneous electromagnetic wave from living tissue serving as a specimen is utilized as an example of the method not having electromagnetic wave application means and composed only of reception means for receiving a spontaneous electromagnetic wave from living tissue serving as a specimen. The above analysis means is analysis means for detecting infrared rays and far infrared rays generated from a specimen such as a human body, the analysis means not having electromagnetic wave application means and being composed only of the reception means for receiving a spontaneous electromagnetic wave from living tissue serving as a specimen. Furthermore, the above analysis means thermally detects the region and phenomenon of abnormalities of living tissue by means of a method not having the application means of an electromagnetic wave such as infrared rays and composed only of the reception means for receiving a spontaneous electromagnetic wave from living tissue serving as a specimen. Since the amount of signals from the analysis means not having the application means of an electromagnetic wave such as infrared rays and composed only of the reception means for receiving a spontaneous electromagnetic wave from living tissue serving as a specimen is large, reduction processing and control are performed through the first-stage digital signal processor (DSP).

The large-scale digital signal processor (DSP) serves as a central unit for analyzing and synthesizing the signals from the analysis means provided with the electromagnetic wave application means and the electromagnetic wave reception means and the signals from the analysis means not having electromagnetic wave application means and composed only of the reception means for receiving a spontaneous electromagnetic wave from living tissue serving as a specimen, and for performing image processing. Computational processing is performed by an image processing chip and software such that the region and the phenomenon can be determined through an image or a signal.

The medium-scale digital signal processor (DSP) performs processing for outputting, as an image, the signals from the large-scale digital signal processor (DSP).

In order to detect a dynamic part in a specimen, the motion is detected by measurement of Doppler effect. Examples of the dynamic part include a heart, a blood vessel, a bloodstream, and a peristaltic motion of a stomach and an intestine.

As an example, by evaluating the flow of blood into an organ, obstruction of the organ and vascularization associated with a tumor can be detected.

Application to the determination of the morphology and the position of a tumor is available by detecting new blood vessels.

An abnormal part (affected part) of a specimen is detected by use of a method for multi-viewing through the use of two or more sensors.

The performance of a synthesized image has performance capable of displaying an abnormal part by applying a color thereto.

As for the resolution, a synthesized image has a high resolution of 0.05 x 0.05 mm² when, for example, application from multiple radiation sources (for example, three radiation sources) is performed on an area of 5 x 5 m² at a radiation angle of 1 degree by use of a solid high frequency oscillation tube in order to avoid interference.

Since this method has high resolution as mentioned above, initial cancer can be detected. In addition, the region of an affected part can be identified at any place (for example, irrespective of the inner wall of intestine or the outer wall of intestine) in a specimen.

### Example 2

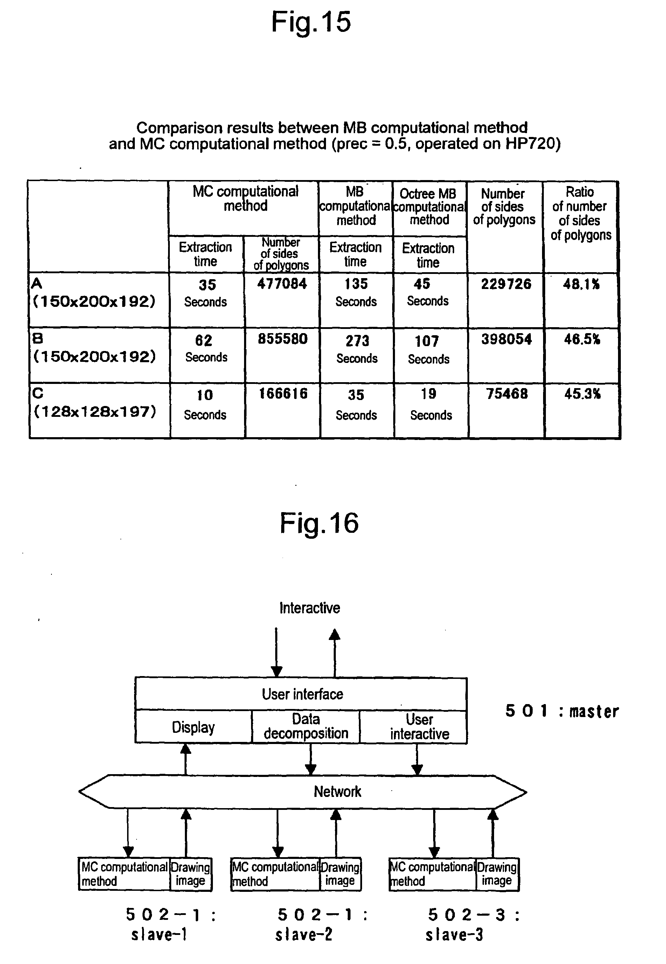

Fig. 15 is an example showing processing time when various computational methods for extracting an isosurface are applied to various measurement subjects. An MC method stands for a Marching Cube method and is a classical computational method. This is a method for drawing the shape of an isosurface of a measurement subject by finding voxels having the same intensity among adjacent voxels.

The structure of the computation is simple. However, the amount of the computation is huge, and thus a computer is required to have high performance.

An MB method stands for a Marching Boxes method. This is a computational method for drawing an isosurface by dividing an original solid body composed of solid data into groups of smaller unit cubes and sequentially merging a unit cube with an adjacent unit cube which has the same intensity as that of the former unit cube.

An Octree MB method is a computational method for performing detection of an isosurface at high speed, and the original image data is converted to Octree representation. The computational time can reduced, and high speed processing can be achieved.

Fig. 15 shows the computational time when each of the MB method, the MC method, and the Octree MB method was applied to subjects A, B, and C. In the MB method and the Octree MB method, the number of sides of polygons could be reduced to 40% or more to less than 50% of that of the MC method. It was found that the computation time of the MB method was larger than the computation time of the MC method for all the measurement subjects. In addition, when the Octree MB method was employed, the computational time could be reduced to nearly the same level as that of the MC Method.

### Example 3

Fig. 16 is a block diagram illustrating the structure of a computational method performed by the inventors and constructed on a UNIX (registered trademark) network to which computers such as SUN Sparc 1 are connected. The method is referred to as a DMC method (Distributed Marching Cubes method).

A program is divided to a master side 501 and slave sides 502. In the master side 501, a user interface, data collection, data decomposition, and the like are performed. In the slave sides 502, computation for an isosurface is performed, and the results are returned to the master side.

The master 501 determines the number of the slaves 502 to be employed according to the size of data. The slave 502 performs the MC method and a Phong model and returns the processing results to the master 501. The master 501 removes hidden surfaces by means of a z-buffer method and displays an image on a display.

In this Example, the MC method was employed as an extraction computation method for an isosurface, and the Phong model, which is a simple gloss model, was employed as a gloss model. Furthermore, the z-buffer method was employed as hidden surface removal which is a method for performing display by removing a portion in the back side of a solid body from a screen. Moreover, in Fig. 16, the number of the slaves was three, and thus slaves 502-1, 502-2, and 502-3 ware employed. The above computational methods are only exemplary and are not limited in the present invention.

The Phong model is a model which is characterized in that a smooth curved surface having a portion highlighted by lighting is formed by use of three characteristics, i.e., scattering diffusion, mirror reflection, and environmental light irradiation. The z-buffer method is a method in which a Z value (the depth from a point of view) of a plane to be displayed is accumulated for each pixel. Each surface is projected onto a perspective projection surface, and the Z value of the surface is stored in the position of pixels occupied by the surface. At this time, when another surface is already stored, the Z values are compared, and the information of a surface having a smaller Z value is stored.

### Example 4

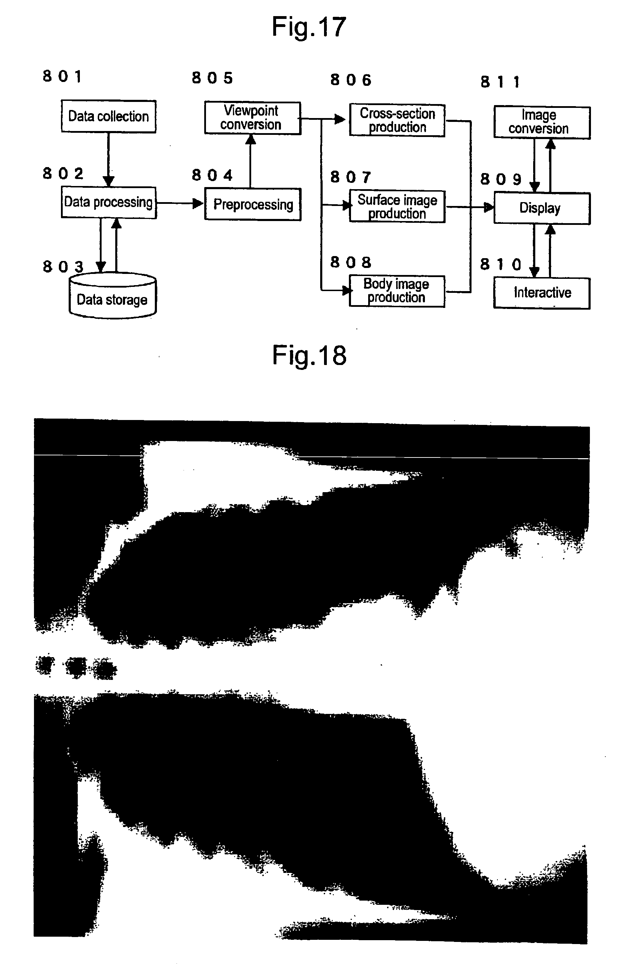

Fig. 17 is an example of a flowchart of visualization of medical imaging. This is referred to as a VOMI system.

Raw data from radar or the like is collected into a data collection unit 801. The data is stored in a data storage 803 or is read from the data storage 803 through a data processing unit 802.

The data storage 803 is a hard disk of a computer, a flexible disk, a CD-R, a magneto optical disc, or a large capacity storage device.

The data proceeds to a preprocessing unit 804 and is subjected to processing such as noise removal.

In a viewpoint conversion unit 805, the setting of the angle for observing a subject or the part of a cross-section of the subject is made.

By means of user input means such as a mouse and a keyboard, the point of view can be changed, and instructions can be provided for displaying from different angles.

Subsequently, branching into three steps is performed in accordance with need. One or two or more steps can be selected in accordance with need.

In a slice production unit 806, computation for drawing a cross-section of an observing region of the subject is performed. A slice of an arbitrary surface is produced from three-dimensional data.

In a surface image producing unit 807, computation for drawing a three-dimensional shape of a part (such as a specific organ or a tumor) of the subject is performed.

Computation for a volume, statistical analysis, two-dimensional and three-dimensional shape measurement can be performed for the region of the subject.

In a three-dimensional image production unit 808, computation for producing semi-translucent three-dimensional image having gradation is performed.

In a display unit 809, an image computed in 806, 807, and 808 is displayed on a display, a printer, a film, or the like.

In an interactive unit 810, a user provides instructions to a computer to set an angle of an image to be displayed, expansion/retraction, a threshold value for determining which organ or affected part is drawn, or the like.

In an image conversion unit 811, the image is converted according to the instructions provided by the interactive unit 810.

### Example 5

Fig. 18 is a schematic diagram of a main portion of a human body, showing an example of gray scale tree-dimensional image display by means of the radar medical diagnostic method and apparatus according to the present invention.

As has been described, the problems are solved by the radar medical diagnostic method and apparatus according to the present invention. That is:
In contrast to an ultrasonic echo diagnostic method and apparatus, the microwave and also infrared rays employed in the radar medical diagnostic method and apparatus according to the present invention provide much higher resolution, and thus a resolution down to several µm can be obtained.

Since the radar medical diagnostic method and apparatus according to the present invention do not employ radioactive rays employed in X-ray tomography and an apparatus therefor (X-ray CT) and positron emission tomography and an apparatus therefor (PET), there is no risk of radioactive damage to an organism such as a human body.

As described in Example 1, the radar medical diagnostic method and apparatus according to the present invention have shorter diagnostic time than X-ray tomography and an apparatus therefor (X-ray CT), a magnetic resonance method and apparatus (MRI), and positron emission tomography and an apparatus therefor (PET).

The radar medical diagnostic method and apparatus according to the present invention do not require a large-scale and expensive apparatus such as an X-ray generator in X-ray tomography and an apparatus therefor (X-ray CT), a magnetic field generator and a gamma ray detector in a magnetic resonance method and apparatus therefore (MRI). These radar medical diagnostic method and apparatus can be formed only of a simple and small antenna or a sensor and a digital signal processor which is an electric circuit, and thus can be produced at low cost.

### INDUSTRIAL APPLICABILITY

The present invention can be employed not only in an electromagnetic wave medical diagnosis but also in a nondestructive diagnosis of an organized body or the like such as a crack or the state of steel bars in a structural body and a material body such as concrete or a tunnel, and in a nondestructive diagnosis in geology, in an underground object diagnosis such as water vein prospecting and the state of piping in a stratum and underground, and in other various nondestructive diagnoses in physics.

## Claims

1. A method for acquiring bioinformation, comprising
a step of preparing in advance a first database by classifying and organizing, for each of components of an organism, millimeter-wave band electromagnetic wave characteristics of a surface of the organism and an inside of the organism which characteristics are contained in transmission electromagnetic wave data and reflection electromagnetic wave data measured with a millimeter-wave band electromagnetic wave applied to the organism,
a step of preparing in advance a second database by classifying and organizing, for each of the components of the organism, electromagnetic wave spontaneous radiation characteristics of the organism which characteristics are contained in spontaneous radiation electromagnetic wave data measured with no electromagnetic wave applied to the organism,
a step of acquiring, based on data in the first database, actual condition information of components of a subject organism from transmission electromagnetic wave data and reflection electromagnetic wave data measured with a millimeter-wave band electromagnetic wave applied to the subject organism, and
a step of acquiring, based on data in the second database, actual condition information of the components of the subject organism from spontaneous radiation electromagnetic wave data measured with no electromagnetic wave applied to the subject organism.

2. The method for acquiring bioinformation according to claim 1,
wherein any one of the steps of preparing the first or second database comprises a step of adding, to the data in the database, meridian point-meridian preset positional information in which a position of a meridian point and a meridian of the organism is represented by positional relation relative to the components of the organism, and
wherein the steps of acquiring the actual condition information of the components of the subject organism comprise a step of determining an actual condition position of the meridian point and the meridian of the subject organism by means of:
the meridian point-meridian preset positional information added to the data of the first or second database;
the transmission electromagnetic wave data and the reflection electromagnetic wave data measured with the millimeter-wave band electromagnetic wave applied to the subject organism; and
the spontaneous radiation electromagnetic wave data measured with no millimeter-wave band electromagnetic wave applied to the subject organism.

3. The method for acquiring bioinformation according to claim 2,
wherein any one of the steps of preparing the first or second database comprises a step of adding, to the data in the first or second database, normal state meridian point-meridian positional range information in which a positional range of the meridian point and the meridian of an organism in a normal state is represented by positional relation relative to the components of the organism, and
wherein the steps of acquiring the actual condition information of the components of the subject organism comprise a step of determining whether or not the position of the meridian point and the meridian of the subject organism is within a normal value range by means of: the actual condition position of the meridian point and the meridian, the actual condition position being obtained in the step of determining the actual condition position of the meridian point and the meridian of the subject organism; and the normal state meridian point-meridian positional range information.

4. The method for acquiring bioinformation according to any of claims 1 to 3,
wherein the millimeter-wave band electromagnetic wave to be applied has a bandwidth of 1.5 GHz or more and has at least an electromagnetic wave component having a wavelength of 6 mm to 14 mm within the bandwidth, and
wherein the millimeter-wave band electromagnetic wave characteristics of the surface of the organism and the inside of the organism which characteristics are contained in the first database and/or the electromagnetic wave spontaneous radiation characteristics of the organism which characteristics are contained in the second database includes at least characteristics for an electromagnetic wave having a wavelength of 6 mm to 14 mm.

5. The method for acquiring bioinformation according to any of claims 1 to 3,
wherein the millimeter-wave band electromagnetic wave to be applied is generated through a plurality of repeated electromagnetic pulses which are continuous in time,
wherein a pulse time width of the electromagnetic pulses is 0.1 to 10 nanoseconds, and
wherein a repeated time interval of the plurality of electromagnetic pulses is 0.1 to 10 microseconds.

6. The method for acquiring bioinformation according to claim 5, wherein a pulse quiescent interval between the plurality of repeated electromagnetic pulses is 100 times to 10,000 times of the pulse time width of the electromagnetic pulses.

7. The method for acquiring bioinformation according to any of claims 1 to 3, wherein the millimeter-wave band electromagnetic wave to be applied is generated at different fixed positions or at a position varying with time,
wherein the method comprises an encryption step of, when the millimeter-wave band electromagnetic wave is applied, encrypting generation position information to superpose the generation position information on the electromagnetic wave to be applied, and
wherein the transmission electromagnetic wave data and the reflection electromagnetic wave data measured with the millimeter-wave band electromagnetic wave applied to the subject organism are decrypted based on the encryption method to add the decrypted generation position information to the millimeter-wave band electromagnetic wave characteristics of the surface of the organism and the inside of the organism.

8. An apparatus for acquiring and displaying bioinformation, comprising:
a first database in which millimeter-wave band electromagnetic wave characteristics of a surface of an organism and an inside of the organism which characteristics are contained in transmission electromagnetic wave data and reflection electromagnetic wave data measured with a millimeter-wave band electromagnetic wave applied to the organism are classified and organized for each of components of the organism;
a second database in which electromagnetic wave spontaneous radiation characteristics of the organism which characteristics are contained in spontaneous radiation electromagnetic wave data measured with no electromagnetic wave applied to the organism are classified and organized for each of actual conditions of components of the organism;
millimeter-wave band electromagnetic wave radiation means for radiating a millimeter-wave band electromagnetic wave toward a subject organism;
first reception means for receiving an electromagnetic wave from the subject organism irradiated with the millimeter-wave band electromagnetic wave;
second reception means for receiving an electromagnetic wave spontaneously radiated from the subject organism;
first organism actual condition information acquisition means for acquiring actual condition information of the components of the subject organism based on data obtained by the first reception means and data in the first database;
second organism actual condition information acquisition means for acquiring actual condition information of the components of the subject organism based on data obtained by the second reception means and data in the second database; and
means for displaying bioinformation based on the data in the first and second databases and information obtained through the first and second organism actual condition information acquisition means.

9. The apparatus for acquiring and displaying bioinformation according to claim 8,
wherein the first or second database contains data for meridian point-meridian preset positional information in which a position of a meridian point and a meridian of the organism is represented by positional relation relative to the components of the organism,
wherein the first or second organism actual condition information acquisition means for acquiring the actual condition information of the components of the subject organism comprises means for determining an actual condition position of the meridian point and the meridian of the subject organism by means of:
the meridian point-meridian preset positional information contained in the data in the first or second database;
the transmission electromagnetic wave data and the reflection electromagnetic wave data measured with the millimeter-wave band electromagnetic wave applied to the subject organism; and
the spontaneous radiation electromagnetic wave data measured with no millimeter-wave band electromagnetic wave applied to the subject organism; and
wherein the means for displaying bioinformation comprises means for displaying the actual condition position of the meridian point and the meridian, the actual condition position being obtained by the means for determining the actual condition position of the meridian point and the meridian of the subject organism.

10. The apparatus for acquiring and displaying bioinformation according to claim 9,
wherein the data in the first or second database contains data for normal state meridian point-meridian positional range information in which a positional range of the meridian point and the meridian of an organism in a normal state is represented by positional relation relative to the components of the organism;
wherein the first or second organism actual condition information acquisition means for acquiring the actual condition information of the components of the subject organism comprises determination means for determining whether or not the position of the meridian point and the meridian of the subject organism is within a normal range by means of: the actual condition position of the meridian point and the meridian, the actual condition position being obtained by the means for determining the actual condition position of the meridian point and the meridian of the subject organism; and the normal state meridian point-meridian positional range information; and
wherein the means for displaying bioinformation comprises means for displaying determination made by the determination means for determining whether or not the position of the meridian point and the meridian of the subject organism is within a normal range.

11. The apparatus for acquiring and displaying bioinformation according to any of claims 8 to 10,
wherein the millimeter-wave band electromagnetic wave to be applied has a bandwidth of 1.5 GHz or more and has at least an electromagnetic wave component having a wavelength of 6 mm to 14 mm within the bandwidth,
wherein the millimeter-wave band electromagnetic wave characteristics of the surface of the organism and the inside of the organism which characteristics are contained in the first database and/or the electromagnetic wave spontaneous radiation characteristics of the organism which characteristics contained in the second database included at least characteristics for an electromagnetic wave having a wavelength of 6 mm to 14 mm,
wherein the millimeter-wave band electromagnetic wave radiation means has a radiation source which radiates an electromagnetic wave containing at least an electromagnetic wave component having a wavelength of 6 mm to 14 mm; and
wherein the first reception means has a receiver which receives an electromagnetic wave containing at least an electromagnetic wave component having a wavelength of 6 mm to 14 mm.

12. The apparatus for acquiring and displaying bioinformation according to any of claims 8 to 10,
wherein the millimeter-wave band electromagnetic wave to be applied is generated through a plurality of repeated electromagnetic pulses which are continuous in time,
wherein a pulse time width of the electromagnetic pulses is 0.1 to 10 nanoseconds, and
wherein a repeated time interval of the plurality of electromagnetic pulses is 0.1 to 10 microseconds.

13. The apparatus for acquiring and displaying bioinformation according to claim 12, wherein a pulse quiescent interval between the plurality of repeated pulses is 100 times to 10,000 times of the pulse time width of the electromagnetic pulses.

14. The apparatus for acquiring and displaying bioinformation according to any of claims 8 to 10,
wherein the millimeter-wave band electromagnetic wave radiation means and the first reception means are transmission/reception means by means of a synthetic aperture radar, transmits/receives a plurality of transmission or reflection electromagnetic waves having phases shifted from each other, and receives from the subject organism an electromagnetic wave which is equivalent to an electromagnetic wave obtained from reception means having a large apparent aperture.

15. The apparatus for acquiring and displaying bioinformation according to any of claims 8 to 10,
wherein the millimeter-wave band electromagnetic wave radiation means is composed of a plurality of fixed radiation sources radiating a millimeter-wave band electromagnetic wave from respective different fixed positions toward an organism and also has electromagnetic wave encryption means for encrypting information of the fixed position of the radiation sources and superposing the information on an electromagnetic wave radiated from the radiation sources, and
wherein the first reception means also has cipher decryption means for decrypting received electromagnetic wave data based on an encryption method employed for encrypting and superposing the information of the fixed position.

16. The apparatus for acquiring and displaying bioinformation according to any of claims 8 to 10,
wherein the millimeter-wave band electromagnetic wave radiation means is a moving radiation source mounted on moving means and radiating a millimeter-wave band electromagnetic wave toward an organism from different positions while moving and also has electromagnetic wave encryption means for encrypting positional information at the time of radiation and superposing the positional information on an electromagnetic wave radiated from the moving radiation sources, and
wherein the first reception means also has cipher decryption means for decrypting received electromagnetic wave data based on an encryption method employed for encrypting and superposing the positional information at the time of electromagnetic wave radiation.

17. An apparatus for acquiring and displaying bioinformation, the apparatus being **characterized in that**
the meridian point-meridian actual condition position display means of claim 9 comprises:
means for constructing, by use of the meridian point serving as a point and the meridian serving as a line, three-dimensional data with regard to each of the meridian point-meridian preset positional information and the actual condition information of the measured meridian point and the measured meridian; and
means for converting the three-dimensional data to two-dimensional display using a point of view.
